# EUROPEAN PATENT APPLICATION

(11) **EP 1 340 494 A1**
(43) Date of publication of application: **03.09.2003**
(21) Application number: 01993393.6
(22) Date of filing: 09.11.2001
(51) Int. Cl.: A61K 9/127, A61K 47/30, B01J 13/04

(54) **METHOD FOR PREPARING MICROSOME DISPERSION**

(30) Priority: 10.11.2000 JP 2000344459
(71) Applicant: JAPAN SCIENCE AND TECHNOLOGY CORPORATION, Kawaguchi-shi, Saitama 332-0012 (JP)
(72) Inventor: TSUCHIDA, Eishun, Nerima-ku, Tokyo 177-0053 (JP); TAKEOKA, Shinji, Setagaya-ku, Tokyo 158-0094 (JP); SOU, Keitaro, Toshima-ku, Tokyo 171-0031 (JP); ENDO, Taro, Tama-shi, Tokyo 206-0034 (JP); NAITO, Yoshiyasu, Yokohama-shi, Kanagawa 244-0801 (JP)
(74) Representative: Calamita, Roberto
(86) International application number: JP0109828
(87) International publication number: WO02038128

(57) **Abstract**

A method for preparing a vesicle dispersion wherein a water-soluble substance is encupsulated in the vesicle, which comprises successively performing: a step of dispersing at least one lipid in an aqueous medium to pre-construct a vesicle dispersion, a step of drying the pre-constructed vesicles to obtain dried vesicles, a step of re-dispersing the dried vesicles in an aqueous solution of the water-soluble substance, and a step of passing the resultant vesicle dispersion through a filter. The method enables the efficient and simple preparation of safe vesicles with a useful substance in high concentration encupsulated therein, with a narrow size distribution, in high yield.

## Description

### Technical Field

The invention of the present application relates to a simple and efficient method for producing a vesicle dispersion of uniform particle size, by effectively encapsulating useful substances such as drugs, physiologically active substances and hemoglobin into vesicles. More particularly, the invention of the present application relates to a method for producing a vesicle dispersion with shortened preparation time and increased yield, that enables the controlling of particle size without the use of organic solvents or surfactants, and enables the mass production of safe vesicle dispersions useful in the fields of medicaments, cosmetics and food.

### Background Art

Vesicles and their dispersions encapsulating useful substances in its inner aqueous phase are important in various fields such as medicaments, cosmetics and food. In particular. the regulation of particle size and number of layers, improvement of encapsulation efficiency and increase of vesicle yield are essential goals for the large scale production of vesicular products. In addition, although the safety of the products is also an essential condition in all of these fields, when utilized as medicaments such as intravenous preparations, it is particularly required that the particle size and the amount of useful substances encapsulated therein are strictly standardized.

However, in aqueous medium generally used for *in vivo* application, there was a problem that the lipid components that construct bilayer membranes are difficult to disperse, and form multi-lamellar vesicles with a wide size distribution. Thus, it was necessary to find a means for regulating the particle size, and to improve the encapsulation efficiency.

Previously, as a method for encapsulating substances into vesicles, various methods such as the dispersion of lipid in an aqueous solution of the desired substance by sonication, forced stirring (homogenizer) or vortex mixing have been reported and practiced. However, these methods had low encapsulation efficiencies, and the size distributions of the resulting vesicles were wide.

Further, as a method suitable for encapsulating high-molecular-weight substances in high encapsulation efficiency, a freeze-thawing method, wherein lipid is dispersed in an aqueous solution of the substance by mechanical stirring, and repeating freezing and thawing to obtain a vesicle dispersion, is known. However, there was a problem that when the solute concentration was high, the effect of freeze-thawing could not be obtained due to the chyoprotective effects of the solute. (The inventors of the present application attempted the freeze-chawing of a vesicle dispersion of highly concentrated hemoglobin solution (35 g/dL), but no change was perceived in the particle size distribution and encapsulation efficiency; hence, this method was confirmed to be ineffective.)

On the other hand, as a method with relatively high encapsulation efficiency that enables controlling of particle size, various methods such as the organic solvent injecting method, wherein lipid solution dissolved in a volatile organic solvent is injected into an aqueous solution of the desired substance, after which the organic solvent is evaporated to obtain a vesicle dispersion; the surfactant removing method wherein a surfactant is removed from a mixed micelle of surfactants and lipids by dialysis; and the reverse phase evaporation method, wherein a lipid is dissolved in an organic solvent immiscible with water. after which a small amount of an aqueous medium is added to form a w/o emulsion by sonication, followed by the removal of the organic solvent under reduced pressure, are known. However, since all of these methods use an organic solvent or a surfactant, denaturation or degradation of the substance to be encapsulated, protein in particular, occurs. Further, there are many problems in terms of safety, because it is difficult to completely remove the organic solvents and surfactants.

Thus, as a method suitable for uniformly controlling the particle size in a vesicle dispersion, an extruding method wherein a vesicle dispersion is permeated through pores of constant size using a French press, a pressure filter or an extruder (Japanese Patent Provisional Publication No. 61-502452) has been reported. However, when such extruding method is applied to a system in which lipid is dispersed in a hemoglobin solution of high concentration, the rate of permeation is dramatically reduced, and clogging of the filter tends to occur, necessitating the frequent exchanging of filters, hence, creating new problems such as high running cost and complication of operation steps. For this reason, the extruding method could not be called a suitable method for the large scale production of vesicle dispersions. Hitherto, various methods for obtaining dispersions of vesicles containing high concentrations of hemoglobin, without the use of filters have been studied. Examples are: the method for preparing hemoglobin vesicles by dispersing powdered lipid mixed with surfactants in a hemoglobin solution and removing the surfactant; the method for preparing hemoglobin vesicles by dispersing powdered lipid in a hemoglobin solution, dehydrating, and redispersing the lipid; and the method of obtaining hemoglobin vesicles with their particle sizes controlled to an extent by passing a dispersion of mixed lipid in hemoglobin solution through a small gap at high pressure.

Further, as a method which does not use organic solvents or surfactants, or include a step of drying the substance, and which is thus suitable for encapsulating unstable substances such as protein, the method of forming a vesicle in an aqueous medium, removing the aqueous medium from the vesicle dispersion to obtain a dried vesicle, and dispersing this dried vesicle in an aqueous solution of the substance intended to be encapsulated has been known (JP-B No. B-505882). However, very little substances can be encapsulated by the mere hydration of such dried vesicles, and reconstruction by forced stirring, microfluidization (microfluidizer) or sonication is necessary, which makes the controlling of particle size difficult. Furthermore, it is known that vesicle dispersions obtained by dispersing vesicles in an aqueous solution by the organic solvent injecting method, freeze-drying the resulting vesicle dispersion to obtain a mixed lipid, and dispersing the lipid in this aqueous solution, do not cause filter-clogging during extrusion (Japanese Patent Application Preliminary Publication (JP-A) No. 9-87168). However, because the method uses organic solvents, from the viewpoint of safety, it is not a suitable method for preparing intravenously injectable preparations.

Therefore, conditions for the preparation of vesicles that take into account the particle size, encapsulation efficiency and filter permeability of vesicles have not been realized.

The inventors of the present application have taken into consideration the fact that strictly controlled particle size and nontoxicity is required in order to use vesicle dispersions with useful substance such as hemoglobin encapsulated therein as intravenously injectable preparations, and extensively studied the optimal conditions for an extrusion method that enables controlling of particle size without the addition of surfactants and organic solvents.

In the method for preparing hemoglobin vesicles by dispersing dried mixed lipid in a hemoglobin solution of high concentration and purity, and successively permeating this dispersion through filters of uniform pore diameters, the high viscosity of the hemoglobin solution itself and the hemoglobin denatured during operation often causes the rate of permeation to decrease, leading to the clogging of filters, as described above. For this reason, filter permeation of the dispersion was time-consuming, and when the membrane area was increased for efficiency, the amount of dispersion that was retained in the pores of the filter increases, leading to reduced yield. Furthermore, since the dispersion is normally permeated successively through filters of different pore size, the type of filters needed are various, and filter exchange becomes troublesome, leading to further decrease in yield and increase in production cost.

### Disclosure of Invention

The invention of the present application was accomplished in view of the aforementioned circumstances, and the object of the present invention is to overcome the problems of the prior art, and to provide a highly efficient method for preparing vesicles of uniform particle size at a high yield, which enables the safe and simple encapsulation of substances in high concentration and purity.

### Brief Description of Drawings

Fig. 1 shows the rate of filter permeation for the hemoglobin vesicle dispersion prepared by the method of the present invention described in the Example; and
Fig. 2 shows the rate of filter permeation of the hemoglobin vesicle dispersion prepared by a previously reported method, described in the Example.

### Best Mode for carrying Out the Invention

In order to solve the aforementioned problems, the invention of the present application firstly provide a method for producing a vesicle dispersion wherein a water-soluble substance is encapsulated in a vesicle and the vesicle size is controlled, which comprises successively performing: a step of dispersing one or more lipids into an aqueous medium for the pre-construction of vesicles; a step of drying the pre-constructed vesicles to obtain dried vesicles; a step of redispersing the dried vesicles into an aqueous solution of a water-soluble substance; and a step of permeating the resulting vesicle dispersion through filters.

The invention of the present application secondly provides the above method for producing a vesicle dispersion. which further comprises one or more freeze-thawing steps between the steps of dispersing one or more lipids in an aqueous medium and the step of drying the pre-constructed vesicle to obtain dried vesicles.

Thirdly, the invention of the present application provides a method for producing a vesicle dispersion, wherein one of the lipids in the aforementioned first or second invention is a polyethylene glycol-type lipid. Further, fourthly, the invention of the present application provides a method for producing a vesicle dispersion, wherein the concentration of the polyethylene glycol-type lipid is in the range of 0.01 to 1 mol%.

In addition, the invention of the present application provides fifthly, any one of the above-described methods for producing a vesicle dispersion, wherein the drying of the vesicles to obtain dried vesicles is performed by freeze-drying; and sixthly, any one of the above-described methods for producing a vesicledispersion, wherein the drying of the vesicles to obtain dried vesicles is performed by spray-drying.

Seventhly, the invention of the present application also provides any of the above-described methods for producing a vesicle dispersion, wherein the water-soluble substance is selected from the group consisting of hemoglobin and stroma-free hemoglobin; and eighthly, the invention of the present application provides the method for producing a vesicle dispersion, wherein the concentration of the aqueous solution of hemoglobin or etroma-free hemoglobin is in the range of 10 to 50 g/dL.

In the method for producing a vesicle dispersion *of* the present invention, the membrane lipid of the vesicles may be one or more lipids selected from various amphiphilic molecules that form bilayer membranes in aqueous solvents. Preferable examples are natural or synthetic saturated phospholipids, unsaturated phospholipids, and combinations thereof.

As saturated phospholipids, natural phospholipids such as hydrogenated egg yolk lecithin and hydrogenated soybean lecithin, and their derivatives, as well as dimyristoylphosphatidylcholine, dipalmitoylphosphathidylcholine and distearoylphosphatidylcholine are exemplified. Further, as unsaturated phospholipids, phospholipid polymers containing polymerizing groups such as egg yolk lecithin, soybean lecithin, 1,2-bis(2,4-octadecadienoyl)-sn-glycero-3-phoephocholine, and 1,2-bis-(8,10,12-octadecatrienoyl)-sn-glycero-3-phosphochol ine are exemplified. Here. the phospholipid polymer may have a non-polymerizable long chain; examples of the non-polymerizable long chain include straight or branched alkyl groups, acyl groups, non-polymerizable alkenyl groups and non-polymerizable alkenoyl groups, with 2 to 24 carbons.

As the membrane lipid of the vesicle, mixed lipids containing a polyethylene glycol-type lipid is especially preferable. The polyethylene glycol chain is preferable since it effectively inhibits vesicle size change and aggregation in the steps following the dispersion of the dried vesicle in an aqueous solution, and can prevent the decrease in filter permeability and clogging of filter often caused by the aggregation of vesicles when performing extrusion. The content of the polyethylene glycol-type lipid is preferably 0.01 to 1 mol%, more preferably 0.1 to 0.3 mol% of the mixed lipid. When the content of the polyethylene glycol-type lipid is less than 0.1 mol%, the aggregation inhibiting effect is weakened. On the other hand, when the content of the polyethylene glycol-type lipid is larger than 0.3 mol%. the efficiency of a water-soluble substance encapsulation tends to be reduced due to the volume exclusion effect of the polyethylene glycol chain extending in the inner phase of the vesicle. Further, the molecular weight of polyethylene glycol is preferably around 2,000 to 12,000.

In the method for producing a vesicle dispersion of the present invention, the membrane lipid of the vesicle may contain a negatively charged lipid, preferable examples of which are diacylphosphatidylglycerol, diacylphosphatidic acid, diacylphosphatidylinositol, diacylphosphatidylserine and fatty acid. Here, the content of the negatively charged lipid is not particularly limited; 1 to 50 mol% is preferable, and 5 to 20 mol% is more preferable. When the content of the negatively charged lipid is less than 1 mol%, the encapsulation efficiency is decreased and aggregation of the vesicles tends to occur. When the content is larger than 50 mol%. the vesicle membrane may become unstable, and thus, is not preferable.

Further, in the method for producing a vesicle dispersion of the present invention, the lipid component used as the membrane lipid of the vesicle may contain a stabilizing agent. Preferable examples of such stabilizing agents are sterols; specific examples include ergosterol and cholesterol, of which cholesterol is preferable. The cholesterol content is not particularly limited, but in order to effectively stabilize the vesicle membrane, 20 to 60 mol% is preferable.

In the method for producing a vesicle dispersion of the present invention, as a "pre-construction" step, one or more of the above-described lipids are dispersed in an aqueous medium, and vesicles are formed in advance. In this pre-construction step, vesicles may be obtained by, for example, adding an aqueous medium to a mixed lipid powder, thereby hydrating and swelling the lipid powder, after which the lipid is dispersed by allowing to stand still, or by using a vortex mixer, a mechanical stirrer. a sonicator, a homogenizer, a microfluidizer, or a high pressure extrusion machine (extruder), or by freeze-thawing. Among such methods, the freeze-thawing method is preferable since it can effectively reduce the number of layers and remarkably improve permeability. The method of pre-constructing vesicles by dispersing lipid in an aqueous medium is not limited to these methods, but other methods such as the organic solvent injection method, the surfactant removing method, the reverse phase evaporation method and the organic solvent pellet evaporation method are not suitable because it is difficult to completely remove the toxic residues.

Through intensive studies by the present inventors, it has been revealed that the size of the vesicles obtained in the preconstruction step is reduced to about 1.3rd to 4th its size in the steps that follow. Therefore, in the preparation step, it is preferable that the particle size of the vesicles is made to be about 1.3 to 4 times that of the desired vesicle product. The final size of the resulting vesicles can vary depending on the use of the vesicle dispersion product, and is not particularly limited. For example, when a hemoglobin-containing vesicle dispersion is used as an intravenously injectable preparation, it is preferable that the final particle size of the vesicle product is 70 nm to 300 nm.

In order to control the particle size of the vesicles to some extent during the above-described pre-construction step, various methods may be applied. For example, a multi-lamellar vesicle of 0.5 to 300 µm is obtained by adding a commercially available lipid powder to an aqueous medium in a concentration of 1 to 5 g/dL. and allowing it to stand at room temperature. When this vesicle dispersion is treated with a vortex mixer for about 5 minutes, multi-lamellar vesicles of 0.3 to 3 µm is obtained; by treating the vesicle dispersion with a probe-type sonicator at a temperature higher than the phase transition temperature for 5 minutes, a vesicle with a particle size of 20 to 200 nm is obtained. In addition, by permeating the multi-lamellar vesicle obtained by incubating at room temperature through a high-pressure extruder, vesicles of an arbitrary particle size with a narrow size distribution consistent with the pore size of the filter can be obtained. For example, by using a filter with a pore size of 0.03 to 3 µm for a lipid concentration of about 1 to 10 g/dL, the particle size can be adjusted by the pore size of the last filter through which the Vesicles are to be permeated.

The vesicle dispersion prepared by the aforementioned method may be fractionated by a variety of methods in order to further decrease the distribution of the particle size. For example, ultracentrifugation and gel filtration may *be* considered. The particle size of the thus prepared vesicle may be measured by various known methods. For example, dynamic light scattering method and electron microscopic method may be applied.

In the method for producing a vesicle dispersion of the present invention, as the aqueous medium, pure water, aqueous solutions and buffer solutions may be used. The aqueous medium can be appropriately selected depending on the use of the vesicle dispersion; water for injection and physiological saline, which may be safely applied to the living body are especially preferable.

In the method for producing a vesicle dispersion of the present invention, the pre-constructed vesicles are dried to obtain dried vesicles. Here, the drying method is not particularly limited and may include methods such as drying under reduced pressure, freeze-drying, spray-drying and cracking. In order to retain the vesicle structure even after the removal of the aqueous medium. the freeze-drying method wherein the dispersion is subjected to rapid freezing followed by removal of moisture, and the spray-drying method wherein the dispersion is rapidly dried after being sprayed are preferable. Of course, the means of drying is not limited to these methods, as long as the vesicle structure is retained.

Next, in the method for producing a vesicle dispersion of the present invention, the dried vesicles obtained by drying the prepared vesicles are added to an aqueous solution of the water-soluble substance that is tobe encapsulated in the vesicle. Since the dried vesicles obtained in the preconstruction step are orientated with the hydrophilic groups facing outward, they may be completely dispersed in the aqueous solution of the water-soluble substance in a short time even without vigorous stirring or heating.

Here, as the water-soluble substance, for example, synthetic substances such as various medicaments and precursors of medicaments, natural substances such as hemoglobin and enzyme, as well as extracts from plants may be considered. For example, when a hemoglobin solution is used, the resulting vesicle dispersion may be preferable as an artificial oxygen carrier. Examples of such hemoglobin solution include a stroma-free hemoglobin solution obtained by hemolyzing human-derived or cow-derived red blood cells by conventional methods, and removing only the stroma component by centrifugation or ultrafiltration; a purified hemoglobin solution obtained by isolating hemoglobin from the above solution; and a recombinant hemoglobin solution that has been concentrated to 10 g/dL or more by ultrafiltration. In order to supply a sufficient amount of oxygen as an oxygen carrier to living tissues, the hemoglobin concentration should preferably be 20 to 50 g/dL.

Further, the water-soluble substance that is to be encapsulated into the vesicle is not limited to one substance, and may be a combination of various substances. For example, when a hemoglobin solution is encapsulated, organic phosphorus compounds such as inositol phosphate and pyridoxal 5'-phosphate may be added to adjust the oxygen affinity of hemoglobin. Alternatively, as a hemoglobin-reducing agent, thiols such as cysteine, homocysteine and glutathione, as well as water-soluble vitamins such as ascorbic acid, maybe added. Further, addition of catalase or superoxide dismutase as oxygen radical scavengers may be considered, too.

In the method for producing a vesicle of the present invention, when the pre-constructed dried vesicles are redispersed in an aqueous solution of a water-soluble substance, the particle size tends to increase slightly. Although such an increase is within 30% of the original vesicle size and does not have a large influence, when polyethylene glycol type lipids are used as the membrane lipid of the vesicle, as described above, the polyethylene glycol chain acts as a protecting agent, so that very little change in particle size occurs.

In the method for producing a vesicle dispersion of the present invention, following the dispersion of the aforementioned dried vesicles in an aqueous solution of a water-soluble substance, the resulting vesicle dispersion is permeated through an isopore membrane filter under high pressure. By this extrusion method, encapsulation of the water-soluble substance into the vesicle proceeds effectively, while the vesicle size is uniformized.

As the filter used in this step, as long as it is water-resistant and has a pore size corresponding to or larger than the desired vesicle size, any single filter or combination thereof is applicable. For example, EXTRUDER (registered trademark) (trade name, manufactured by Nichiyu Liposome) with a membrane area of 3.1 cm² may be used. The amount of the sample dispersion to be applied may be selected appropriately, depending on the membrane area etc. In addition, the pressure applied is not particularly limited either; a pressure at which a suitable permeation rate is maintained may be selected as long as the membrane is not ruptured. Generally, a pressure of 20 kg/cm² or lower is used.

As described above, the size of the preconstructed vesicle is reduced to 1.3rd to 4th for the final size of the vesicles obtained by filter permeation. Therefore, the particle size of the vesicles obtained in the preconstruction step must be determined with this point taken into consideration. When the vesicles with a particle size smaller than 1.3rd of the final vesicles are formed in the preparation step, vesicle reconstruction does not occur because the vesicle pass through the filter in the filter permeation step, and the encapsulation efficiency is not increased. On the other hand, when vesicles with a particle size larger than 4th of the final vesicles are formed in the preconetruction step, the filter permeation rate decreases and the filter is easily clogged, causing an increase in the number of steps; hence, it is not suitable for the large scale production of a vesicle dispersion.

The method for producing a vesicle dispersion of the present invention is as described above; however, other steps such as freeze-thawing, stirring and heating may further be included to the aforementioned steps of vesicle pre-construction, drying, redispersion of the dried vesicles to an aqueous solution of a water-soluble substance, and permeation of the resulting vesicle dispersion through a filter. In addition, any remaining water-soluble substance that is not encapsulated into the vesicle may be removed by ultracentrifugation, gel filtration or ultrafiltration membrane. Further, in the filter permeation step, the filter is not limited to one; filters of equal sized pores may be used repeatedly, or various filters with different-sized pores may be used to successively decrease the vesicle size.

Embodiments of the present invention will be described in further detail by the following Examples in reference to the attached drawings. Of course, the present invention is not limited to the following examples, and it goes without saying that various modifications of the details are possible.

### Examples

In the following Examples and Comparative Examples, the encapsulation efficiency of hemoglobin was determined as the numerical value (A/B) obtained by dividing the weight of hemoglobin (A) in the hemoglobin-encapsulating vesicle dispersion obtained by removing any unencapsulated hemoglobin by the total lipid weight (B). Therefore, it can be said that the larger the value of A/B, the higher the hemoglobin encapsulation efficiency is.

The weight of hemoglobin can be calculated by the cyanomethemoglobin method, and the total lipid weight can be calculated from phosphorus quantitation by the permanganate ashing method or from cholesterol quantitation by the enzyme measuring method; however, herein, a commercially available quantitation kit was used.

The filter permeability was determined by introducing 5 mL of the sample dispersion into EXTRUDER (registered trademark)(trade name, manufactured by Nichiyu Liposome) with a membrane area of 3.1 cm², receiving the permeated solution in a scaled cylinder while pressurizing at a constant pressure (20 kg/cm²) and recording the increase of the liquid surface on video tape.

### <Example 1>

A mixed lipid consisting of 144 mg (0.2 mmol) of dipalmitoylphosphatidylcholine, 76 mg (0.2 mmol) of cholesterol and 29 mg (0.04 mmol) of dipalmitoylphosphatidylglycerol was dispersed in 5 mL of water for injection as membrane lipids, and stirred at 25°C to obtain a multi-lamellar vesicle dispersion. This dispersion was subjected to three cycles of freeze-thawing wherein the dispersion was frozen with liquid nitrogen and thawed at 25°C. to obtain a dispersion of 500 nm vesicles. This vesicle dispersion was spray-dried to obtain dried vesicles. The lyophilized vesicles were added to 5 mL (35 g/dL) of a hemoglobin solution, which was stirred at 25°C for 2 hours. This was then subjected to an EXTRUDER (registered trademark)(trade name, manufactured by Nichiyu Liposome), and successively extruded throughacetylcellulose filters (manufactured by Fuji Photo Film Co., Ltd.) of pore sizes 3.0 µm, 0.8 µm, 0.65 µm, 0.45 µm, 0.30 µm and 0.22 µm at 14°C under a pressure of 20 kg/cm².

No clogging occurred in any of the filters, and the permeability was good.

The remaining hemoglobin was removed by subjecting the prepared sample to 3 cycles of ultracentrifugation (100,000 g, 60 min). The particle size of the thus obtained hemoglobin vesicles was determined to be 252 ± 52 nm using a dynamic light scattering apparatus (COULTER N4SD).

Using a commercially available phosphorus quantitation kit and hemoglobin quantitation kit. phosphorus quancitation and hemoglobin quancitation were performed. The total lipid weight was determined from phosphorus quantitation, and the hemoglobin weight was divided by the total lipid weight, whereby the hemoglobin/total lipid ratio was found to be 1.7.

### <Comparative Example 1>

After the vesicles were pre-constructed by the method described in Example 1. the vesicles were precipitated by ultracentrifugation (300,000 g, 60 min) without spray-drying; the supernatant was removed and the remaining vesicles were disperse in 5 mL (35 g/dL) of hemoglobin solution.

This was subjected to an EXTRUDER (registered trademark) (trade name, manufactured by Nichiyu Liposome), and successively extruded through acetylcellulose filters (manufactured by Fuji Photo Film Co., Ltd.) of pore sizes 3.0 µm, 0.8 µm, 0.65 µm, 0.45 µm, 0.30 µm and 0.22 µm at 14°C under a pressure of 20 kg/cm².

In all of the filters, the permeability was good.

The sample was then subjected to three cycles of ultracentrifugation (100,000 g, 60 min) to remove the remaining hemoglobin. The particle size of the thus obtained hemoglobin vesicles was determined as 250 ± 50 nm using a dynamic light scattering apparatus (COULTER N4SD).

Using a commercially available phosphorus quantitation kit and hemoglobin quantitation kit, phosphorus quantitaion and hemoglobin quantitaion were performed. The total lipid weight was obtained from phosphorus quantitation, and the hemoglobin weight was divided by the total lipid weight, whereby the hemoglobin/total lipid ratio was found to be 0.8.

Hemoglobin/total lipid ratios obtained in Example 1 and Comparative Example 1 are shown in Table 1.

**Table 1**

| Sample | Filter permeability | Final particle size (nm) | Hemoglobin/Total lipid ratio (wt/wt) |
|---|---|---|---|
| Ex. 1 | Good | 255±52 | 1.7 |
| Comp. Ex. 1 | Good | 250±50 | 0.8 |

From Table 1, it can be seen chat, in the production of a vesicle dispersion, the filter permeability is good when the step of obtaining dried vesicles is not included (Comparative Example 1), as is the case when the step of obtaining dried (lyophilized) vesicles is included (Example 1). However, it was shown that the encapsulation efficiency by extrusion is reduced by not including the step of obtaining dried vesicles. This may be caused by the aqueous medium used during the pre-construction step, remaining in the inner phase of the vesicle.

### <Example 2>

A mixed lipid consisting of 432 mg (0.6 mmol) of dipalmitoylphosphatidylcholine, 228 mg (0.6 mmol) of cholesterol and 87 mg (0.12 mmol) of dipalmitoylpbosphatidylglycerol was dispersed in 15 mL of water for injection, as membrane lipids. and stirred at 25°C to obtain a multi-lamellar vesicle dispersion. This dispersion was separated into three 5 mL portions and subjected to (a) a system wherein particle size adjustment by pre-treatment is not performed, (b) a system wherein the particle size is adjusted to 500 nm by extrusion, and (c) a system wherein the particle size is adjusted to 250 nm by extrusion, which were then frozen with liquid nitrogen, fitted to a lyophilizer, and freeze-dried for 12 hours to obtain lyophilized vesicles.

5 mL (35 g/dL) of a stroma-free hemoglobin solution was added to each sample, and stirred at 14°C for 2 hours to obtain a hemoglobin vesicle dispersion.

This was then subjected co an EXTRUDER (registered trademark) (trade name, manufactured by Nichiyu Liposome) , and successively extruded through acetylcellulose filters (manufactured by Fuji Photo Film Co., Ltd.) of pore sizes 3.0 µm, 0.8 µm, 0.65 µm, 0.45 µm, 0.30 µm and 0.22 µm at 14°C under a pressure of 20 kg/cm².

No clogging occurred in any of the filters, and the permeability was good.

The remaining hemoglobin was removed by subjecting the prepared sample to 3 cycles of ultracentrifugation (100,000 g, 60 min). The particle size of the thus obtained hemoglobin vesicles was determined by a dynamic light scattering apparatus (COULTER N4SD).

Using a commercially available phosphorus quantitation kit and hemoglobin quantitation kit, phosphorus quantitation and hemoglobin quantitation were performed. The total lipid weight was determined from phosphorus quantitation, and the hemoglobin weight was divided by the total lipid weight, whereby the hemoglobin/total lipid ratio was calculated.

The filter permeability, particle size and hemoglobin encapsulation efficiency (hemoglobin/lipid) of the resulting vesicles are shown in Table 2.

**Table 2**

| Sample | Particle size at Preparation (nm) | Filter permeability | Final Particle Size (nm) | Hemoglobin/Total lipid ratio (wt/wt) |
|---|---|---|---|---|
| (a) | 1800±180 | Low | 265±54 | 1.6 |
| (b) | 515± 72 | Good | 256±51 | 1.7 |
| (c) | 258±512 | Excellent | 250±45 | 0.3 |

Since the filter permeability was low when the pre-constructed vesicle size (1800 nm) was 7 times that of the desired vesicle size (265 nm), extrusion had to be performed using a filter with larger pore size. When the pre-constructed vesicle size (258 nm) was one times that of the desired vesicle size (250 nm) (sample(c)), the filter permeability was high, but the vesicle passed through the pores of the filter, causing a reduction in the encapsulation efficiency.

On the other hand, when the pre-constructed vesicle size (515 nm) was 2 times that of the desired vesicle size (256 nm) (sample(b)), good filter permeability and high encapsulation efficiency were obtained.

### <Example 3>

As the vesicle membrane lipid, 144 mg (0.2 mmol) of dipalmitoylphosphatidylcholine, 76 mg (0.2 mmol) of cholesterol, 29 mg (0.04 mmol) of dispalmitoylphosphatidylglycerol and 7 mg (1.3 µmol) of distearoyl-N-monomethoxy-polyethyleneglycol (molecular weight: 5,000)-succinylphosphatidylethanolamine were weighed, and added to a 10 mL flask. 5 mL of benzene was added thereto, and the lipid was completely dissolved under heating. This solution was frozen with liquid nitrogen, fitted to a lyophilizer, and freeze-dried for 12 hours to obtain a white powder. This powder was added to 5 mL of water for injection, and stirred at 25°C to obtain a vesicle dispersion with a vesicle size of 1.8 µm. This dispersion was subjected to four cycles of freeze-thawing, consisting of freezing with liquid nitrogen and thawing at 25°C, whereby a dispersion of 520 nm vesicles was obtained. This dispersion was frozen with liquid nitrogen, fitted to a lyophilizer, and freeze-dried for 15 hours to obtain a white dried vesicles.

5 mL (35 g/dL) of hemoglobin solution was added to the dried vesicles, and stirred at 25°C to obtain a hemoglobin vesicle dispersion. Here, the vesicle size was 540 nm, almost maintaining the size of the pre-constructed vesicle.

This was then subjected to an EXTRUDER (registered trademark)(trade name, manufactured by Nichiyu Liposome), and successively extruded through acetylcellulose filters (manufactured by Fuji Photo Film Co., Ltd.) of pore sizes 3.0 µm, 0.8 µm, 0.65 µm, 0.45 µm, 0.30 µm and 0.22 µm at 14°C under a pressure of 20 kg/cm². The permeation behavior of the dispersion was recorded on video tape, and the time consumed for permeation and the permeated volume of the dispersion were measured.

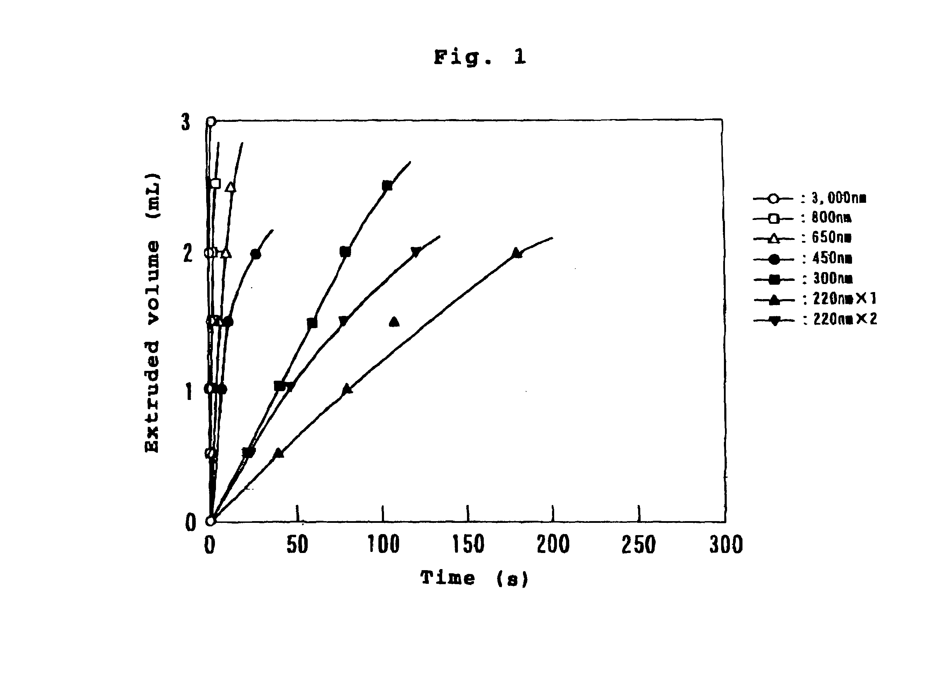

The relationship between the extruded volume and time is shown in Fig.1.

As shown in Fig.1, according to the method for producing a vesicle dispersion of the present invention. a final vesicle size of 250 nm was obtained while maintaining good filter permeability, and hemoglobin could be encapsulated at a hemoglobin/total lipid ratio of 1.7.

### <Comparative Example 2>

After the vesicle was pre-constructed by the method described in Example 3, the vesicle solution was frozen with liquid nitrogen without the freeze-thawing step for particle size control. Then, the frozen vesicle dispersion was put in a lyophilizer, and freeze-dried for 12 hours to Obtain a white powdery dried vesicles.

5 mL (35 g/dL) of hemoglobin solution was added to the dried vesicles, and stirred at 25°C to obtain a hemoglobin vesicle dispersion.

This was then subjected to an EXTRUDER (registered trademark) (trade name, manufactured by Nichiyu Liposome), and successively extruded through acetylcellulose filters (manufactured by Fuji Photo Film Co., Ltd.) of pore sizes 3.0 µm, 0.8 µm, 0.65 µm, 0.45 µm, 0.30 µm and 0.22 µm at 14°C under a pressure of 20 kg/cm². The permeation behavior of the dispersion was recorded on video tape. and the time consumed for permeation and the permeated volume of the dispersion were measured.

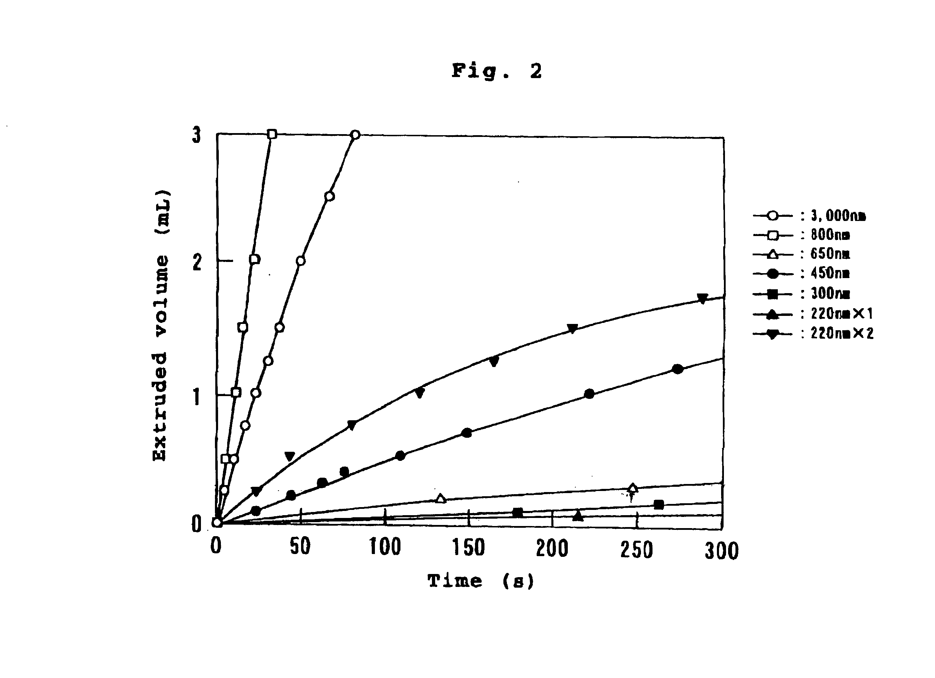

The relationship between the extruded volume and time is shown in Fig.2

Fig.2 shows chat when a conventional method, in which control of a particle size is not performed was used, the permeation rate for each filter was reduced in comparison with Example 3.

### <Example 4>

As the membrane lipid of vesicles, a mixed lipid consisting of 8.64 g (12.0 mmol) of dipalmitoylphosphatidylcholine, 4,56 g (12.0 mmol) of cholesterol, 1.74 g (2.4 mmol) of dipalmitoylphosphatidylglycerol and 0.42 g (78 µmol) of distearoyl-N-monomethoxy-polyethyleneglycolsuccinylphosphat idylethanolamine was dispersed in 300 mL of aqueous sodium hydroxide ([sodium hydroxide] - 8 mM), and stirred at 25°C to obtain a multi-lamellar vesicle dispersion.

This dispersion was subjected to four cycles of freeze-thawing, consisting of freezing with liquid nitrogen and thawing at 25°C, whereby a dispersion of 520 nm vesicles was obtained. This dispersion was frozen with liquid nitrogen, fitted to a lyophilizer, and freeze-dried for 15 hours to obtain a white dried vesicles.

300 mL (35 g/dL) of hemoglobin solution was added to the dried vesicles, and stirred at 14°C for 2 hours to obtain a vesicle dispersion. This dispersion was separated into two 150 mL portions, each subjected to (d) a system wherein the dispersion is added to Lemolino (trade name, manufactured by Millipore Corporation) with a membrane area of 45.3 cm², and successively extruded through acetylcellulose filters (manufactured by Fuji Photo Film Co., Ltd.) of pore sizes 0.65 µm, 0.45 µm, 0.30 µm and 0.22 µm at 14°C under a pressure of 20 kg/cm², (e) a system where the dispersion is added to a microfluidizer, and subjected to 10 cycles of 10,000 psi.

Each sample was subjected to three cycles of ultracentrifugation (100,000 g, 60 min), to remove the unencapsulated hemoglobin. The particle size of the thus obtained hemoglobin vesicle dispersion was measured using a dynamic light scattering apparatus (COULTER N4SD). Using a commercially available phosphorus quantitation kit and hemoglobin quantitation kit, phosphorus quantitation and hemoglobin quantitation were performed, and the hemoglobin weight was divided by the total lipid weight, whereby the hemoglobin/total lipid ratio was calculated.

**Table 3**

| Sample | Means of size control | Final particle size (nm) | Hemoglobin/Total lipid ratio (wt/wt) |
|---|---|---|---|
| (d) | Extrusion (Lemolino) | 255± 42 | 1.8 |
| (e) | Microfluidizer | 221±112 | 1.4 |

From Table 3, it was shown that, when the particle size is adjusted by microfluidizer, the encapusulation efficiency becomes relatively low, and the size distribution wide; thus, it would be necessary to add a size fractionation step, which complicates the production steps and reduces the yield.

On the other hand, it was confirmed that when the extrusion method is used, the size distribution becomes narrow and the encapsulation efficiency high.

### <Example 5>

As a vesicle membrane lipid, 144 mg (0.2 mmol) of dipalmitoylphosphatidylcholile, 76 mg (0.2mmol) of cholesterol, 29 mg (0.04 mmol) of dipalmitoylphoephatidylglycerol and 7 mg (1.3 µmol) of distearoyl-N-monomethoxy-polyetyleneglycol (molecular weight: 5000)-succinylphosphosphatidylethanolamine were weighed, and added to a 10 mL flask. 5 mL of benzene was added thereto, and the lipid was completely dissolved under heating. This solution was frozen with liquid nitrogen, fitted to a lyophilizer, and freeze-dried for 12 hours to obtain a white powder. This powder was added to 25 mL of water for injection. and stirred at 25°C to obtain a multi-lamellar vesicle dispersion. This dispersion was subjected to four cycles of freeze-thawing, consisting of freezing with liquid nitrogen and thawing at 25°C, whereby a dispersion of 500 nm vesicles was obtained. This dispersion was frozen with liquid nitrogen, fitted to a lyophilizer, and freeze-dried for 15 hours to obtain a white dried vesicles.

5 mL (35 g/dL) of hemoglobin solution was added to the dried vesicles, and stirred at 25°C to obtain a hemoglobin vesicle dispersion. Here, the vesicle size was 520 nm. almost maintaining the size of the vesicle before. This was then subjected to an EXTRUDER (registered trademark)(trade name, manufactured by Nichiyu Liposome). and successively extruded through acetylcellulose filters (manufactured by Fuji Photo Film Co., Ltd.) of pore sizes 3.0 µm, 0.45 µm, 0.30 µm and 0.22 µm at 14°C under a pressure of 20 kg/cm². The permeability through all of the filters were good.

### <Comparative Example 3>

As a vesicle membrane lipid, 144 mg (0.2 mmol) of dipalmitoylphosphatidylcholine, 76mg (0.2mmol) or cholesterol, and 29 mg (0.04 mmol) of dipalmitoylphosphatidylglycerol were weighed, and added to a 10 mL flask. 5 mL of benzene was added thereto, and the lipid was completely dissolved under heating. This solution was frozen with liquid nitrogen, fitted to a lyophilizer, and freeze-dried for 12 hours to obtain a white powder. This powder was added to 25 mL of water for injection, and stirred at 25°C to obtain a multi-lamellar vesicle dispersion. This dispersion was subjected to four cycles of freeze-thawing, consisting of freezing with liquid nitrogen and thawing at 25°C, whereby a dispersion of 520 nm vesicles was obtained. This dispersion was frozen with liquid nitrogen, fitted to a lyophilizer, and freeze-dried for 15 hours to obtain a white dried vesicles.

5 mL (35 g/dL) of hemoglobin solution was added to the dried vesicles, and stirred at 25°C to obtain a hemoglobin vesicle dispersion. Here, the vesicle size was 650 nm, slightly larger than the pre-constructed vesicles. This was then subjected to an EXTRUDER (registered trademark) (trade name, manufactured by Nichiyu Liposome), and successively extruded through acetylcellulose filters (manufactured by Fuji Photo Film Co., Ltd.) of pore sizes 3.0 µm, 0.45 µm, 0.30 µm and 0.22 µm at 14°C under a pressure of 20 kg/cm². The permeability was good for all of the filters. However, when compared to the result where a polyethyleneglycol type lipid was used as the membrane lipid, the permeation rate was reduced when the pore size of the filter was 0.45 µm or smaller.

### <Comparative Example 4>

As a vesicle membrane lipid, 144 mg (0.2 mmol) of dipalmitoylphosphatidylcholine. 76 mg (0.2 mmol) of cholesterol, 29 mg (0.04 mmol) of dipalmitoylphosphatidylglycerol and 61 mg (11.3 µmol) of distearoyl-N-monomethoxy-polyethyleneglycol (molecular weight: 5,000)-succinylphosphatidylechanolamine were weighed, and added to a 10 mL flask. 5 mL of benzene was added thereto, and the lipid was completely dissolved under heating. This solution was frozen with liquid nitrogen, fitted to a lyophilizer, and freeze-dried for 12 hours to obtain a white powder. This powder was added to 25 mL of water for injection, and stirred at 25°C to obtain a multi-lamellar vesicle dispersion. This dispersion was subjected to four cycles of freeze-thawing, consisting of freezing with liquid nitrogen and thawing at 25°C, whereby a dispersion of 500 nm vesicles was obtained. This dispersion was frozen with liquid nitrogen, fitted to a lyophilizer, and freeze-dried for 15 hours to obtain a white dried vesicles.

5 mL (35 g/dL) of hemoglobin solution was added to the dried vesicles, and stirred at 25°C to obtain a hemoglobin vesicle dispersion. Here, the vesicle size was 500 nm, maintaining the size of the vesicle before. This was then subjected to an EXTRUDER (registered trademark)(trade name, manufactured by Nichiyu Liposome), and successively extruded through acetylcellulose filters (manufactured by Fuji Photo Film Co., Ltd.) of pore sizes 3.0 µm, 0.45 µm, 0.30 µm and 0.22 µm at 14°C under a pressure of 20 kg/cm². Although the filter permeability was excellent, reduction in the hemoglobin encapsulation efficiency was observed.

The vesicle size at pre-construccion, the vesicle size after dispersion into a hemoglobin solution, the filter permeability and the hemoglobin/total lipid ratio (wt/wt) for Example 5 and Comparative Examples 3 and 4 are shown in Table 4.

**Table 4**

| Sample (Polyethyle neglycol-type lipid) | Size at Pre-construction (nm) | Size after dispersion in hemoglobin solution (nm) | Filter Permeability | Hemoglobin/ Total lipid ratio (wt/wt) |
|---|---|---|---|---|
| Example 5 (0.3mol%) | 500 | 520 | Excellent | 1.9 |
| Comparative Example 3 (0mol%) | 520 | 650 | Good | 1.8 |
| Comparativo Example 4 (2.5mol%) | 500 | 500 | Excellent | 1.3 |

### Industrial Applicability

As described in detail above, according to the present invention, a method that enables the efficient encapsulation of high concentrations of useful substances, and the safe and simple preparation of vesicles with uniform particle size in high yield is provided.

## Claims

1. A method for producing a vesicle dispersion wherein a water-soluble substance is encapsulated in a vesicle and the vesicle size is controlled, which comprises successively performing:
a step of dispersing one or more lipids into an aqueous medium to pre-construct Vesicles;
a step of drying the pre-constructed vesicles to obtain dried vesicles;
a step of re-dispersing the dried veeicles into an aqueous solution of a water-soluble substance; and
a seep of permeating the resulting vesicle dispersion through one or more filters.

2. The method for producing a vesicle dispersion of claim 1, which further comprises one or more freeze-thawing steps between the step of dispersing one or more lipids in an aqueous medium and the step of drying the pre-constructed vesicles to obtain dried vesicles.

3. The method for producing a vesicle dispersion of claim 1 or 2, wherein one of the lipids is a polyethylene glycol-type lipid,

4. The method for producing a vesicle dispersion of claim 3, wherein the concentration of the polyethylene glycol-type lipid is in the range of 0.01 to 1 mol%.

5. The method for producing a vesicle dispersion of any one of claims 1 to 4, wherein the dried vesicles are obtained by freeze-drying the vesicles.

6. The method for producing a vesicle dispersion of any one of claims 1 to 4, wherein the dried vesicles are obtained by spray-drying the vesicles.

7. The method for producing a vesicle dispersion of any one of claims 1 to 6, wherein the water-soluble substance is selected from the group consisting of hemoglobin and stroma-free hemoglobin.

8. The method for producing a vesicle dispersion of claim 7, wherein the concentration of the aqueous solution of hemoglobin or stroma-free hemoglobin is in the range of 10 to 50 g/dL.
